# EUROPEAN PATENT SPECIFICATION

(11) **EP 2 521 790 B1**
(45) Date of publication and mention of the grant of the patent: **22.09.2021**
(21) Application number: 10828637.8
(22) Date of filing: 12.05.2010
(51) Int. Cl.: C12P 1/04, C12P 5/00, C12P 7/00, C12P 9/00, C12P 11/00

(54) **BIOLOGICAL AND CHEMICAL PROCESS UTILIZING CHEMOAUTOTROPHIC MICROORGANISMS FOR THE CHEMOSYNTHETIC FIXATION OF CARBON DIOXIDE AND/OR OTHER INORGANIC CARBON SOURCES INTO ORGANIC COMPOUNDS, AND THE GENERATION OF ADDITIONAL USEFUL PRODUCTS**
BIOLOGISCHES UND CHEMISCHES VERFAHREN MIT CHEMOAUTOTROPHEN MIKROORGANISMEN ZUR CHEMOSYNTHETISCHEN FIXIERUNG VON KOHLENDIOXID UND/ODER ANDEREN ANORGANISCHEN KOHLENSTOFFQUELLEN IN ORGANISCHEN VERBINDUNGEN SOWIE ERZEUGUNG VON ZUSÄTZLICHEN NÜTZLICHEN PRODUKTEN
PROCÉDÉ BIOLOGIQUE ET CHIMIQUE UTILISANT DES MICROORGANISMES CHIMIOTROPHES POUR LA PRODUCTION DE COMPOSÉS ORGANIQUES PAR FIXATION CHIMIO-SYNTHÉTIQUE DE DIOXYDE DE CARBONE ET/OU D'AUTRES SOURCES DE CARBONE INORGANIQUE, ET GÉNÉRATION DE PRODUITS ADDITIONNELS UTILES

(30) Priority: 06.11.2009 US 613550
(43) Date of publication of application: 14.11.2012
(73) Proprietor: Kiverdi, Inc., Berkeley, CA 94704 (US)
(72) Inventor: REED, John, Sacramento CA 95814 (US)
(74) Representative: Bridle, Andrew Barry
(86) International application number: PCT/US2010/001402
(87) International publication number: WO 2011/056183

(56) References cited:
- EP-A1- 1 264 895
- EP-A2- 0 131 220
- WO-A1-98/00558
- WO-A2-02/08438
- JP-A- H06 169 783
- US-A- 4 859 588
- US-A- 5 173 429
- US-A- 5 593 886
- US-A- 5 593 886
- US-A- 5 821 111
- US-B2- 7 285 402
- Aharon Oren: "Chemolithotrophy" In: "Encyclopedia of Life Sciences", 15 September 2009 (2009-09-15), John Wiley & Sons, Ltd, Chichester, UK, XP055084607, ISBN: 978-0-47-001590-2 DOI: 10.1002/9780470015902.a0021153, * the whole document *
- None

## Description

### FIELD OF THE INVENTION

The present invention falls within the technical areas of biofuels, bioremediation, carbon capture, carbon dioxide-to-fuels, carbon recycling, carbon sequestration, energy storage, and renewable/alternative and/or low carbon dioxide emission sources of energy. Specifically the present invention involves in certain aspects a unique use of biocatalysts within a biological and chemical process to fix carbon dioxide and/or other forms of inorganic carbon into organic chemical products through chemosynthesis. In addition certain embodiments of the present invention involve the production of chemical co-products that are co-generated through chemosynthetic reaction steps and/or non-biological reaction steps as part of an overall carbon capture and conversion process. The present invention can enable the effective capture of carbon dioxide from the atmosphere or from a point source of carbon dioxide emissions for the production of liquid transportation fuel and/or other organic chemical products, which can help address greenhouse gas induced climate change and contribute to the domestic production of renewable liquid transportation fuels without any dependence upon agriculture.

### BACKGROUND OF THE INVENTION

The amazing technological and economic progress achieved in the past 100 years has largely been powered by fossil fuels. However the sustainability of this progress is now coming into question, both due to the rise in greenhouses gases caused by fossil fuel combustion, and the increasing scarcity of fossil fuel resources.

Hydrogen which can be generated through a number of different inorganic renewable energy technologies including solar, wind, and geothermal has been proposed as a replacement for hydrocarbon fuels. But hydrogen has its own set of problems including most notably problems with storage. Ironically the best chemical storage medium for hydrogen both in terms of volumetric and gravimetric energy densities is quite possibly hydrocarbons such as gasoline, suggesting that the quest for hydrogen fuel may simply lead full circle back to hydrocarbons.

Biofuels are a promising type of renewable hydrocarbon generally made through the capture and conversion of CO₂ into organic matter by photosynthetic organisms. Since the current transportation fleet and infrastructure is designed for fossil fuels with similar properties to biofuels, it can be more readily be adapted to biofuels, than to inorganic energy storage products such as hydrogen or batteries. A further advantage of biofuels, and hydrocarbons in general, is that they have some of the highest volumetric and gravimetric energy densities found for any form of chemical energy storage - substantially higher than that achieved with current lithium battery and hydrogen storage technologies. However, biofuels produced through photosynthesis have their own set of problems.

Most biofuel currently produced relies on agriculture. The heavy requirements of large scale agricultural biofuel projects for arable land, fresh water, and other resources required for plant growth have been blamed for rapidly increasing food prices and loss of natural habitat [The Price of Biofuels: The Economics Behind Alternative Fuels, Technology Review, January/ February 2008].

As an alternative to higher order plants, photosynthetic microorganisms such as algae and cyanobacteria are being looked at for applications converting CO₂ into biofuels or other organic chemicals [Sheehan et al, 1998, "A Look Back at the U.S. Department of Energy's Aquatic Species Program―Biodiesel from Algae"]. Algal and cyanobacterial technologies benefit from relatively high growth rates, far surpassing higher order plants in their rate of carbon fixation per unit standing biomass. In one promising application of algal technology a high rate of carbon fixation and biomass production is achieved by directing a concentrated stream of CO₂, such as is emitted from industrial point sources, through algae containing bioreactors [Bayless et al. U.S. patent # 6,667,171].

Technologies based on photosynthetic microbes share the drawback common to all photosynthetic systems in that carbon fixation only happens with light exposure. If the light level is deficient, an algal system can actually become a net producer of CO₂ emissions. A bioreactor or pond used to grow photosynthetic microbes such as algae must have a high surface area to volume ratio in order to allow each cell to receive enough light for carbon fixation and cell growth. Otherwise light blockage by cells on the surface will leave cells located towards the center of the volume in darkness - turning them into net CO₂ emitters. This high surface area to volume ratio needed for efficient implementation of the algal and cyanobacterial technologies generally results in either a large land footprint (ponds) or high material costs (bioreactors). The types of materials that can be used in algal bioreactor construction is limited by the requirement that walls lying between the light source and the algal growth environment need to be transparent. This requirement restricts the use of construction materials that would normally be preferred for use in large scale projects such as concrete, steel and earthworks.

In addition to the biological CO₂ fixation processes that have been discussed, there are also fully chemical processes for fixing CO₂ to organic compounds (LBNL Helios; LANL Green Freedom; Sandia Sunshine to Petrol; PARC). The fully chemical technologies are currently hindered by the catalysts that are needed for the relatively complicated reaction of CO₂ to fixed carbon, especially C₂ and longer hydrocarbons.

Chemoautotrophic microorganisms are known that catalyzing the carbon fixation reaction without photosynthesis. The chemosynthetic reactions performed by chemoautotrophs for the fixation of CO₂, and other forms of inorganic carbon, to organic compounds, is powered by potential energy stored in inorganic chemicals, rather than by the radiant energy of light [Shively et al, 1998; Smith et al, 1967; Hugler et al, 2005; Hugker et al., 2005; Scott and Cavanaugh, 2007]. Carbon fixing biochemical pathways that occur in chemoautotrophs include the reductive tricarboxylic acid cycle, the Calvin-Benson-Bassham cycle [Jessup Shively, Geertje van Kaulen, Wim Meijer,, Annu. Rev. Microbiol., 1998, 191-230], and the Wood-Ljungdahl pathway [Ljungdahl, 1986; Gottschalk, 1989; Lee, 2008; Fischer, 2008].

Prior work is known relating to certain applications of chemoautotrophic microorganisms in the capture and conversion of CO₂ gas to fixed carbon [United States Patent 4,596,778 "Single cell protein from sulfur energy sources" Hitzman, June 24, 1986], [United States Patent 4,859,588 "Production of a single cell protein", Sublette August 22, 1989], [United States Patent 5,593,886 "Clostridium strain which produces acetic acid from waste gases Gaddy", January 14, 1997], [United States Patent 5,989,513 "Biologically assisted process for treating sour gas at high pH", Rai November 23, 1999]. However, each of these conventional approaches have suffered shortcomings that have limited the effectiveness, economic feasibility, practicality and commercial adoption of the described processes. The present invention in certain aspects addresses one or more of the aforementioned shortcomings.

Chemoautotrophic microorganisms have also been used to biologically convert syngas into C₂ and longer organic compounds including acetic acid and acetate, and biofuels such as ethanol and butanol [Gaddy, 2007; Lewis, 2007; Heiskanen, 2007; Worden, 1991; Klasson, 1992; Ahmed, 2006; Cotter, 2008; Piccolo, 2008, Wei, 2008]; however, in such approaches the feedstock is strictly limited to fixed carbon (either biomass or fossil fuel), which is gasified and then biologically converted to another form of fixed carbon ― biofuel, and the carbon source and energy source utilized in the process come from the same process input, either biomass or fossil fuel, and are completely intermixed within the syngas in the form of H₂, CO, and CO₂. The present inventors have recognized in the context of the present invention that a need exists for processes that do not require any fixed carbon feedstock, only CO₂ and/or other forms of inorganic carbon and/or utilize a carbon source and energy source that are derived from separate process inputs.

It is known in prior art to fix carbon dioxide, using chemoautotrophic species and hydrogen produced from solar power, from a patent, focussing on the production of methane and pharmaceutical products [JP H06 169783].

Several chemoautotrophic species, including Ralstonia, are described in a scientific paper disclosing carbon dioxide conversion to organic carbon in the Calvin cycle [Aharon Oren: "Chemolithotrophy"In: "ENCYCLOPEDIA OF LIFE SCIENCES", 15 September 2009 (2009-09-15), John Wiley & Sons, Ltd, Chichester].

### SUMMARY OF THE INVENTION

In response to a need in the art that the inventors have recognized in making the invention, a novel combined biological and chemical process for the capture and conversion of inorganic carbon to organic compounds that uses chemosynthetic microorganisms for carbon fixation and that is designed to couple the efficient production of high value organic compounds such as liquid hydrocarbon fuel with the capture of CO₂ emissions, making carbon capture a revenue generating process is described.

According to a first aspect of the invention, there is provided a biological and chemical method as defined in the claims appended hereto.

Described herein are biological and chemical processes for the capture and conversion of carbon dioxide and/or other sources of inorganic carbon, into organic compounds comprising: introducing carbon dioxide gas, either alone and/or dissolved in a mixture or solution further comprising carbonate ion and/or bicarbonate ion, into an environment suitable for maintaining chemoautotrophic organisms and/or chemoautotroph cell extracts; and fixing the carbon dioxide and/or inorganic carbon into organic compounds within the environment via at least one chemosynthetic carbon fixing reaction utilizing *Ralstonia sp.;* wherein where the chemosynthetic carbon fixing reaction is driven by chemical and/or electrochemical energy provided by electron donors and electron acceptors that have been generated chemically and/or electrochemically and/or or are introduced into the environment from at least one source external to the environment.

The carbon source may be separated from the energy source in certain embodiments of the present invention which enables it to function as a far more general energy conversion technology than syngas to liquid fuel conversions. This is because the electron donors used in the present invention can be generated from a wide array of different CO₂-free energy sources, both conventional and alternative, while for syngas conversions to biofuel, all the energy stored in the biofuel is ultimately derived from photosynthesis (with additional geochemical energy in the case of fossil fuel feedstock).

In these process steps chemoautotrophic microorganisms perform chemosynthesis to fix inorganic carbon into organic compounds using the chemical energy stored in hydrogen as an electron donor pumped or otherwise provided to the nutrient media. The electron donor is oxidized by electron acceptors in the chemosynthetic reaction. Electron acceptors that may be used at the chemosynthetic reaction step include carbon dioxide and/or oxygen.

The chemosynthetic reaction step or steps of certain inventive processes wherein carbon dioxide and/or inorganic carbon is fixed into organic carbon in the form of organic compounds and biomass can be performed in aerobic, microaerobic, anoxic, anaerobic, or facultative conditions. A facultative environment is considered to be one where the water column is stratified into aerobic layers and anaerobic layers. The oxygen level maintained spatially and temporally in the system will depend upon the chemoautotrophic species used, and the desired chemosynthesis reactions to be performed.

An additional feature of certain embodiments of the present invention regards the source, production, or recycling of the electron donor used by the chemoautotrophic microorganisms to fix carbon dioxide into organic compounds. The electron donor used for carbon dioxide capture and carbon fixation can be produced or recycled in the present invention electrochemically or thermochemically using power from a number of different renewable and/or low carbon emission energy technologies including but not limited to: photovoltaics, solar thermal, wind power, hydroelectric, nuclear, geothermal, enhanced geothermal, ocean thermal, ocean wave power, tidal power.

An additional feature of certain embodiments of the present invention regards the formation and recovery of biochemicals and/or biomass from the chemosynthetic carbon fixation step or steps. These biochemical and/or biomass products can have applications including but not limited to one or more of the following: as a carbon source for large scale fermentations; as a nutrient source for the growth of other microbes or organisms; as feed for animals including but not limited to cattle, sheep, chickens, pigs, or fish; as sources of pharmaceutical, medicinal or nutritional substances; soil additives and soil stabilizers.

### BRIEF DESCRIPTION OF THE FIGURES

Non-limiting embodiments of the present invention will be described by way of example with reference to the accompanying figures, which are schematic and are not intended to be drawn to scale. For purposes of clarity, not every component is labeled in every figure, nor is every component of each embodiment of the invention shown where illustration is not necessary to allow those of ordinary skill in the art to understand the invention. In the figures:
FIG. 1 is a general process flow diagram for one embodiment of this invention for a carbon capture and fixation process;
FIG. 2 is process flow diagram for another embodiment of the present invention with capture of CO₂ performed by hydrogen oxidizing chemoautotrophs resulting in the production of ethanol;
FIG. 3 shows the mass balance calculated for the embodiment of FIG. 2 reacting CO ₂ with H₂ to produce ethanol;
FIG. 4 shows the enthalpy flow calculated for the embodiment of FIG. 2 reacting CO₂ with H₂ to produce ethanol;
FIG. 5 shows the energy balance calculated for the embodiment of FIG. 2 reacting CO₂ with H₂ to produce ethanol;
FIG. 6. is a process flow diagram of a comparative method for the capture of CO₂ by sulfur oxidizing chemoautotrophs and production of biomass and sulfuric acid,;
FIG 7. is a process flow diagram of a comparative method for the capture of CO₂ by sulfur oxidizing chemoautotrophs and production of biomass and sulfuric acid through the chemosynthetic reaction and calcium carbonate via the Muller-Kuhne reaction,;
FIG. 8 is a process flow diagram of a comparative method for the capture of CO₂ by sulfur oxidizing chemoautotrophs and production of biomass and calcium carbonate and recycling of thiosulfate electron donor via the Muller-Kuhne reaction;
FIG. 9 is a process flow diagram of a comparative method for the capture of CO₂ by sulfur and iron oxidizing chemoautotrophs and production of biomass and sulfuric acid using an insoluble source of electron;
FIG. 10 is a process flow diagram of a comparative method for the capture of CO₂ by sulfur and hydrogen oxidizing chemoautotrophs and production of biomass, sulfuric acid, and ethanol using an insoluble source of electron donors,; and
FIG. 11 is a process flow diagram of a comparative method for the capture of CO₂ by iron and hydrogen oxidizing chemoautotrophs and production of biomass, ferric sulfate, carbonate and ethanol using coal or another hydrocarbon to generate electron donors in a process that does not emit gaseous CO₂ emissions.

### DETAILED DESCRIPTION

The present invention provides, in certain embodiments, compositions and methods for the capture and fixation of carbon dioxide from carbon dioxide-containing gas streams and/or atmospheric carbon dioxide or carbon dioxide in liquefied or chemically-bound form through a chemical and biological process. In addition the present invention, in certain embodiments, provides a method for the recovery, processing, and use of the chemical products of chemosynthetic reaction step or steps performed by *Ralstonia sp.* to fix inorganic carbon into organic compounds.

The genus of chemoautotrophic microorganisms that can be used in one or more process steps of the present invention comprises *Ralstonia sp..*

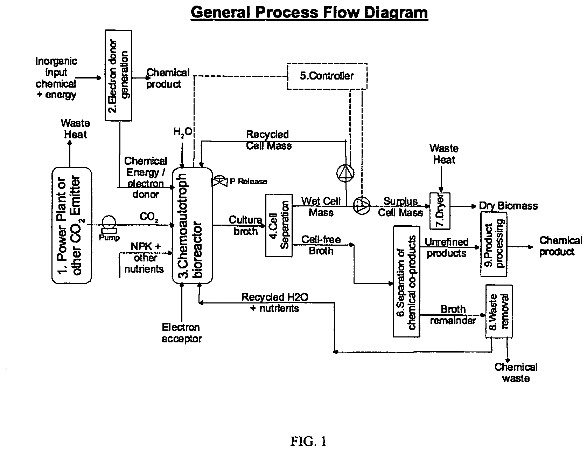

FIG 1 illustrates the general process flow diagram for certain embodiments of the present invention that have a process step for the generation of electron donors suitable for supporting chemosynthesis from an energy input and raw inorganic chemical input; followed by recovery of chemical products from the electron donor generation step; delivery of generated electron donors along with electron acceptors, water, nutrients, and CO₂ from a point industrial flue gas source, into chemosynthetic reaction step or steps that make use of chemoautotrophic microorganisms to capture and fix carbon dioxide, creating chemical and biomass co-products through chemosynthetic reactions; followed by process steps for the recovery of both chemical and biomass products from the process stream; and recycling of unused nutrients and process water, as well as cell mass needed to maintain the chemoautotrophic culture back into the chemosynthetic reaction steps. In the embodiment illustrated in FIG. 1, the CO₂ containing flue gas is captured from a point source or emitter. Electron donors needed for chemosynthesis may be generated from input inorganic chemicals and energy. The flue gas is pumped through bioreactors containing *Ralstonia sp.* along with electron donors and acceptors to drive chemosynthesis and a medium suitable to support a chemoautotrophic culture and carbon fixation through chemosynthesis. The cell culture may be continuously flowed into and out of the bioreactors. After the cell culture leaves the bioreactors the cell mass is separated from the liquid medium. Cell mass needed to replenish the cell culture population at a functional or an optimal level is recycled back into the bioreactor. Surplus cell mass may be dried to form a dry biomass product. Following the cell separation step chemical products of the chemosynthetic reaction may be removed from the process flow and recovered. Then any undesirable waste products that might be present may be removed. Following this, in the illustrated embodiment, the liquid medium and any unused nutrients are recycled back into the bioreactors.

Certain embodiments of the present invention use carbon dioxide emission-free or low-carbon emission and/or renewable sources of power in the production of electron donors including but not limited to one or more of the following: photovoltaics, solar thermal, wind power, hydroelectric, nuclear, geothermal, enhanced geothermal, ocean thermal, ocean wave power, tidal power. In certain embodiments of the present invention that draw upon carbon dioxide emission-free or low-carbon emission and/or renewable sources of power in the production of electron donors, chemoautotrophs function as biocatalysts for the conversion of renewable energy into liquid hydrocarbon fuel, or high energy density organic compounds generally, with CO₂ captured from flue gases, or from the atmosphere, or ocean serving as a carbon source. These embodiments of the present invention can provide renewable energy technologies with the capability of producing a transportation fuel having significantly higher energy density than if the renewable energy sources are used to produce hydrogen gas - which must be stored in relatively heavy storage systems (e.g. tanks or storage materials) - or if it is used to charge batteries which have relatively low energy density. Additionally the liquid hydrocarbon fuel product of certain embodiments of the present invention may be more compatible with the current transportation infrastructure compared to these other energy storage options. The ability of chemoautotrophs to use inorganic sources of chemical energy also enables the conversion of inorganic carbon into liquid hydrocarbon fuels using non-hydrocarbon mineralogical sources of chemical energy, i.e. reduced inorganic minerals (such as hydrogen sulfide, pyrite), which represent a largely untapped store of geochemical energy. Hence certain embodiments of the present invention use mineralogical sources of chemical energy which are pre-processed ahead of the chemosynthetic reaction steps into a form of electron donor and method of electron donor delivery that is suitable or optimal for supporting chemoautotrophic carbon fixation.

The position of the process step or steps for the generation of electron donors in the general process flow of the present invention is illustrated in FIG. 1 by the box 2. labeled "Electron Donor Generation". Electron donors produced in the present invention using electrochemical and/or thermochemical processes known in the art of chemical engineering and/or generated from natural sources include hydrogen.

The present invention uses molecular hydrogen as the electron donor. Hydrogen electron donor may be generated by methods known in to art of chemical and process engineering including but not limited to more or more of the following: through electrolysis of water including but not limited to approaches using Proton Exchange Membranes (PEM), liquid electrolytes such as KOH, high-pressure electrolysis, high temperature electrolysis of steam (HTES).

Certain embodiments of the present invention that use electrical power for the generation of electron donors, receive the electrical power from carbon dioxide emission-free or low-carbon emission and/or renewable sources of power in the production of electron donors including but not limited to one or more of the following: photovoltaics, solar thermal, wind power, hydroelectric, nuclear, geothermal, enhanced geothermal, ocean thermal, ocean wave power, tidal power.

In certain embodiments, the generated electron donors are oxidized in the chemosynthetic reaction step or steps by electron acceptors that include carbon dioxide and/or oxygen.

The position of the chemosynthetic reaction step or steps in the general process flow of the present invention is illustrated in FIG. 1 by the box 3. labeled "Chemoautotroph bioreactor".

At each step in the process where chemosynthetic reactions occur one or more types of electron donor and one or more types of electron acceptor may be pumped or otherwise added to the reaction vessel as either a bolus addition, or periodically, or continuously to the nutrient medium containing chemoautotrophic organisms. The chemosynthetic reaction driven by the transfer of electrons from electron donor to electron acceptor fixes inorganic carbon dioxide into organic compounds and biomass.

In certain embodiments of the present invention electron mediators may be included in the nutrient medium to facilitate the delivery of reducing equivalents from electron donors to chemoautotrophic organisms in the presence of electron acceptors and inorganic carbon in order to kinetically enhance the chemosynthetic reaction step. This aspect of the present invention is particularly applicable to embodiments of the present invention using poorly soluble electron donors such as H₂ gas. The delivery of reducing equivalents from electron donors to the chemoautotrophic organisms for the chemosynthetic reaction or reactions can be kinetically and/or thermodynamically enhanced in the present invention through means including but not limited to: the introduction of hydrogen storage materials into the chemoautotrophic culture environment that can double as a solid support media for microbial growth - bringing absorbed or adsorbed hydrogen electron donors into close proximity with the hydrogen-oxidizing chemoautotrophs; the introduction of electron mediators known in the art such as but not limited to cytochromes, formate, methyl-viologen, NAD+/NADH, neutral red (NR), and quinones into the chemoautotrophic culture media.

The culture broth used in the chemosynthetic steps of certain embodiments of the present invention may be an aqueous solution containing suitable minerals, salts, vitamins, cofactors, buffers, and other components needed for microbial growth, known to those skilled in the art [Bailey and Ollis, Biochemical Engineering Fundamentals, 2nd ed; pp 383-384 and 620-622; McGraw-Hill: New York (1986)]. These nutrients can be chosen to facilitate or maximize *Ralstonia sp.* growth and promote the chemosynthetic enzymatic pathways. Alternative growth environments such as used in the arts of solid state or non-aqueous fermentation may be used in certain embodiments. In certain embodiments that utilize an aqueous culture broth, salt water, sea water, or other non-potable sources of water are used when tolerated by the *Ralstonia sp.* organisms.

The chemosynthetic pathways may be controlled and optimized in certain embodiments of the present invention for the production of chemical products and/or biomass by maintaining specific growth conditions (e.g. levels of nitrogen, oxygen, phosphorous, sulfur, trace micronutrients such as inorganic ions, and if present any regulatory molecules that might not generally be considered a nutrient or energy source). Depending upon the embodiment of the invention the broth may be maintained in aerobic, microaerobic, anoxic, anaerobic, or facultative conditions depending upon the requirements of the *Ralstonia sp.* organisms and the desired products to be created by the chemosynthetic process. A facultative environment is considered to be one having aerobic upper layers and anaerobic lower layers caused by stratification of the water column.

The source of inorganic carbon used in the chemosynthetic reaction process steps of certain embodiments of the present invention includes but is not limited to one or more of the following: a carbon dioxide-containing gas stream that may be pure or a mixture; liquefied CO₂; dry ice; dissolved carbon dioxide, carbonate ion, or bicarbonate ion in solutions including aqueous solutions such as sea water. Carbon dioxide and/or other forms of inorganic carbon may be introduced to the nutrient medium contained in reaction vessels either as a bolus addition or periodically or continuously at the steps in the process where chemosynthesis occurs. In certain embodiments of the present invention, carbon dioxide containing flue gases are captured from the smoke stack at temperature, pressure, and gas composition characteristic of the untreated exhaust, and directed with minimal modification into the reaction vessel(s) where chemosynthesis occurs. Particularly for embodiments where impurities harmful to chemoautotrophic organisms are not present in the flue gas, modification of the flue gas upon entering the reaction vessels may be substantially limited to compression needed to pump the gas through the reactor system and heat exchange needed to lower the gas temperature to one suitable for the microorganisms.

Gases in addition to carbon dioxide that are dissolved into the culture broth of certain embodiments of the present invention may include gaseous electron donors in certain embodiments such as but not limited to hydrogen, carbon monoxide, hydrogen sulfide or other sour gases; and for certain aerobic embodiments of the present invention, oxygen electron acceptor, generally from air (e.g. 20.9% oxygen). The dissolution of these and other gases into solution may be achieved using a system of compressors, flowmeters, and flow valves known to one of skilled in the art of bioreactor scale microbial culturing, that feed into one of more of the following widely used systems for pumping gas into solution: sparging equipment; diffusers including but not limited to dome, tubular, disc, or doughnut geometries; coarse or fine bubble aerators; venturi equipment. In certain embodiments of the present invention surface aeration may also be performed using paddle aerators and the like. In certain embodiments of the present invention gas dissolution is enhanced by mechanical mixing with an impeller or turbine, as well as hydraulic shear devices to reduce bubble size. Following passage through the reactor system holding *Ralstonia sp.* microorganisms which capture the carbon dioxide, the scrubbed flue gas, which is generally comprised primarily of inert gases such as nitrogen, may be released into the atmosphere.

In certain embodiments of the present invention, hydrogen gas is fed to the chemoautotrophic bioreactor either by bubbling it through the culture medium, or by diffusing it through a membrane that bounds the culture medium. The latter method may be safer in certain cases, since hydrogen accumulating in the gas phase can potentially create explosive conditions (the range of explosive hydrogen concentrations in air is 4 to 74.5% and may be avoided in certain embodiments of the present invention).

In certain aerobic embodiments of the present invention that require the pumping of air or oxygen into the culture broth in order to maintain oxygenated levels, oxygen bubbles are injected into the broth at an appropriate or optimal diameter for mixing and oxygen transfer. In one exemplary embodiment, the average diameter of the oxygen bubbles is selected to be about 2 mm, which has been found to be optimal in certain cases *[*Environment Research Journal May/June 1999 pgs. 307-315]. In certain aerobic embodiments of the present invention a process of shearing the oxygen bubbles is used to achieve this bubble diameter as described in U.S. Pat. No. 7,332,077. In certain embodiments, bubble size is controlled to yield values a no larger than 7.5 mm average diameter without substantial slugging.

Additional chemicals to facilitate *Ralstonia sp.* maintenance and growth as known in the art may be added to the culture broth of certain embodiments of the present invention. The concentrations of nutrient chemicals, and particularly the electron donors and acceptors, may be maintained as close as possible to their respective optimal levels for maximum chemoautotrophic growth and/or carbon uptake and fixation and/or production of organic compounds, which varies depending upon the chemoautotrophic species utilized but is known or determinable without undue experimentation to one of skilled in the art of culturing chemoautotrophs.

Along with nutrient levels, the waste product levels, pH, temperature, salinity, dissolved oxygen and carbon dioxide, gas and liquid flow rates, agitation rate, and pressure in the chemoautotrophic culture environment may be controlled in certain embodiments of the present invention as well. The operating parameters affecting chemoautotrophic growth may be monitored with sensors (e.g. dissolved oxygen probe or oxidation-reduction probe to gauge electron donor/acceptor concentrations), and controlled either manually or automatically based upon feedback from sensors through the use of equipment including but not limited to actuating valves, pumps, and agitators. The temperature of the incoming broth as well as incoming gases may be regulated by unit operations such as but not limited to heat exchangers.

Agitation of the culture broth in certain embodiments of the present invention may be provided for mixing and may be accomplished by equipment including but not limited to: recirculation of broth from the bottom of the container to the top via a recirculation conduit; sparging with carbon dioxide plus in certain embodiments electron donor gas (i.e. H₂), and for certain aerobic embodiments of the present invention oxygen or air as well; a mechanical mixer such as but not limited to an impeller (100-1000 rpm) or turbine.

In certain embodiments, the *Ralstonia sp.* microorganism containing nutrient medium is removed from the chemosynthetic reactors partially or completely, periodically or continuously, and is replaced with fresh cell-free medium to maintain the cell culture in exponential growth phase and/or replenish the depleted nutrients in the growth medium and/or remove inhibitory waste products.

The production of useful chemical products through the chemosynthetic reaction step or steps reacting electron donors and acceptors to fix carbon dioxide is a feature of certain embodiments of the present invention. Optimizing the production of a desired chemical product of chemosynthesis may be achieved in certain embodiments of the present invention through control of the parameters in the chemoautotrophic culture environment including but not limited to: nutrient levels, waste levels, pH, temperature, salinity, dissolved oxygen and carbon dioxide, gas and liquid flow rates, agitation rate, and pressure

The high growth rate of *Ralstonia sp.* species enables them to equal or even surpass the highest rates of carbon fixation, and biomass production per standing unit biomass attainable by photosynthetic microbes. Consequently the production of surplus biomass is a feature of certain embodiments of the present invention. Surplus growth of cell mass may be removed from the system to produce a biomass product, and in order to maintain an optimal microbial population and cell density in the chemoautotrophic culture for continued high carbon capture and fixation rates.

Another feature of certain embodiments of the present invention is the vessels used to contain the chemosynthetic reaction environment in the carbon capture and fixation process. The types of culture vessels that can be used in the present invention to culture and grow the chemoautotrophic bacteria for carbon dioxide capture and fixation are generally known in the art of large scale microbial culturing. These culture vessels, which may be of natural or artificial origin, include but are not limited to: airlift reactors; biological scrubber columns; bioreactors; bubble columns; caverns; caves; cisterns; continuous stirred tank reactors; counter-current, upflow, expanded-bed reactors; digesters and in particular digester systems such as known in the prior arts of sewage and waste water treatment or bioremediation; filters including but not limited to trickling filters, rotating biological contactor filters, rotating discs, soil filters; fluidized bed reactors; gas lift fermenters; immobilized cell reactors; lagoons; membrane biofilm reactors; mine shafts; pachuca tanks; packed-bed reactors; plug-flow reactors; ponds; pools; quarries; reservoirs; static mixers; tanks; towers; trickle bed reactors; vats; wells ― with the vessel base, siding, walls, lining, or top constructed out of one or more materials including but not limited to bitumen, cement, ceramics, clay, concrete, epoxy, fiberglass, glass, macadam, plastics, sand, sealant, soil, steels or other metals and their alloys, stone, tar, wood, and any combination thereof. In embodiments of the present invention where the *Ralstonia sp.* microorganisms either require a corrosive growth environment and/or produce corrosive chemicals through the chemosynthetic metabolism corrosion resistant materials may be used to line the interior of the container contacting the growth medium.

Certain embodiments of the present invention will minimize material costs by using chemosynthetic vessel geometries having a low surface area to volume ratio, such as but not limited to substantially cubic, cylindrical shapes with medium aspect ratio, substantially ellipsoidal or "egg-shaped", substantially hemispherical, or substantially spherical shapes, unless material costs are superseded by other design considerations (e.g. land footprint size). The ability to use compact reactor geometries is enabled by the absence of a light requirement for chemosynthetic reactions, in contrast to photosynthetic technologies where the surface area to volume ratio must be large to provide sufficient light exposure.

The *Ralstonia sp.* lack of dependence on light also can allow plant designs with a much smaller footprint than photosynthetic approaches allow. In situations where the plant footprint needs to be minimized due to restricted land availability, certain embodiments of the present invention may use a long vertical shaft bioreactor system for chemoautotrophic growth and carbon capture. A bioreactor of the long vertical shaft type is described in U.S. Pat. Nos. 4,279,754, 5,645,726, 5,650,070, and 7,332,077.

Unless superseded by other considerations, certain embodiments of the present invention may advantageously minimize vessel surfaces across which high losses of water, nutrients, and/or heat may occur, or which potentially permit the introduction of invasive predators into the reactor. The ability to minimize such surfaces , in certain embodiments, is enabled by the lack of light requirements for chemosynthesis.

In certain embodiments of the present invention the *Ralstonia sp.* microorganisms are immobilized within their growth environment. This may be accomplished using any suitable media known in the art of microbial culturing to support colonization by *Ralstonia sp.* microorganisms including but not limited to growing the *Ralstonia sp.* on a matrix, mesh, or membrane made from any of a wide range of natural and synthetic materials and polymers including but not limited to one or more of the following: glass wool, clay, concrete, wood fiber, inorganic oxides such as ZrO₂, Sb₂O₃, or Al₂O₃, the organic polymer polysulfone, or open-pore polyurethane foam having high specific surface area. The *Ralstonia sp.* microorganisms in the present invention may also be grown on the surfaces of unattached objects distributed throughout the growth container as are known in the art of microbial culturing that include but are not limited to one or more of the following: beads; sand; silicates; sepiolite; glass; ceramics; small diameter plastic discs, spheres, tubes, particles, or other shapes known in the art; shredded coconut hulls; ground corn cobs; activated charcoal; granulated coal; crushed coral; sponge balls; suspended media; bits of small diameter rubber (elastomeric) polyethylene tubing; hanging strings of porous fabric, Berl saddles, Raschig rings.

Inoculation of the *Ralstonia sp.* culture into the culture vessel, in certain embodiments, may be performed by methods including but not limited to transfer of culture from an existing *Ralstonia sp.* culture inhabiting another carbon capture and fixation system of the present invention, or incubation from a seed stock raised in an incubator. The seed stock of *Ralstonia sp.* strains, in certain embodiments, may be transported and stored in forms including but not limited to a powder, liquid, frozen, or freeze-dried form as well as any other suitable form, which may be readily recognized by one skilled in the art. When establishing a culture in a very large reactor it may be advantageous in certain cases to grow and establish cultures in progressively larger intermediate scale containers prior to inoculation of the full scale vessel.

The position of the process step or steps for the separation of cell mass from the process stream in the general process flow of the embodiment of the present invention illustrated in FIG. 1 is shown by the box 4. labeled "Cell Separation".

Separation of cell mass from liquid suspension in certain embodiments of the present invention can be performed by methods known in the art of microbial culturing [Examples of cell mass harvesting techniques are given in International Patent Application No. WO08/00558, published Jan. 8, 1998; U.S. Pat. No. 5,807,722; U.S. Pat. No. 5,593,886 and U.S. Pat. No. 5,821,111. ] including but not limited to one or more of the following: centrifugation; flocculation; flotation; filtration using a membranous, hollow fiber, spiral wound, or ceramic filter system; vacuum filtration; tangential flow filtration; clarification; settling; hydrocyclone. In embodiments where the cell mass is immobilized on a matrix it may be harvested by methods including but not limited to gravity sedimentation or filtration, and separated from the growth substrate by liquid shear forces.

In certain embodiments of the present invention, if an excess of cell mass has been removed from the culture, it is recycled back into the cell culture as indicated by the process arrow labeled "Recycled Cell Mass" in FIG 1., along with fresh broth such that sufficient biomass is retained in the chemosynthetic reaction step or steps for continued optimal inorganic carbon uptake and growth or metabolic rate. The cell mass recovered by the harvesting system may be recycled back into the culture vessel using, for example, an airlift or geyser pump. In certain embodiments, the cell mass recycled back into the culture vessel has not been exposed to flocculating agents, unless those agents are non-toxic to the *Ralstonia sp..*

In the present invention, the chemoautotrophic system is maintained, using continuous influx and removal of nutrient medium and/or biomass, in substantially steady state where the cell population and environmental parameters (e.g. cell density, chemical concentrations) are targeted at a substantially constant suitable or optimal level over time. Cell densities may be monitored in certain embodiments of the present invention either by direct sampling, by a correlation of optical density to cell density, or with a particle size analyzer. The hydraulic and biomass retention times can be decoupled so as to allow independent control of both the broth chemistry and the cell density in certain embodiments. Dilution rates may be kept high enough so that the hydraulic retention time is relatively low compared to the biomass retention time, resulting in a highly replenished broth for cell growth. Dilution rates may be set at an appropriate or optimal trade-off between culture broth replenishment, and increased process costs from pumping, increased inputs, and other demands that rise with dilution rates.

To assist in the processing of the biomass product into biofuels or other useful products, the surplus microbial cells in certain embodiments of the invention are broken open following the cell separation step using methods including but not limited to ball milling, cavitation pressure, sonication, or mechanical shearing.

The harvested biomass in certain embodiments of the present invention is dried in the process step or steps of box 7. labeled "Dryer" in the general process flow illustrated in FIG. 1.

Surplus biomass drying may be performed in certain embodiments of the present invention using technologies including but not limited to centrifugation, drum drying, evaporation, freeze drying, heating, spray drying, vacuum drying, vacuum filtration. Heat waste from the industrial source of flue gas may be used in drying the biomass in certain embodiments. In addition the chemosynthetic oxidation of electron donors is exothermic and generally produces waste heat. In certain embodiments of the present invention waste heat can be used in drying the biomass.

In certain embodiments of the invention, the biomass is further processed following drying to aid the production of biofuels or other useful chemicals through the separation of the lipid content or other targeted biochemicals from the chemoautotrophic biomass. The separation of the lipids may be performed by using nonpolar solvents to extract the lipids such as, but not limited to, hexane, cyclohexane, ethyl ether, alcohol (isopropanol, ethanol, etc.), tributyl phosphate, supercritical carbon dioxide, trioctylphosphine oxide, secondary and tertiary amines, or propane. Other useful biochemicals may be extracted in certain embodiments using solvents including but not limited to: chloroform, acetone, ethyl acetate, and tetrachloroethylene.

The broth left over following the removal of cell mass may be pumped to a system for removal of the products of chemosynthesis and/or spent nutrients which may be recycled or recovered to the extent possible, or else disposed of. The position of the process step or steps for the recovery of chemical products from the process stream in the general process flow of the embodiment of present invention illustrated in FIG. 1 is indicated by the box 6. labeled "Separation of chemical products".

Recovery and/or recycling of chemosynthetic chemical products and/or spent nutrients from the aqueous broth solution may be accomplished in certain embodiments of the present invention using equipment and techniques known in the art of process engineering, and targeted towards the chemical products of particular embodiments of the present invention, including but not limited to: solvent extraction; water extraction; distillation; fractional distillation; cementation; chemical precipitation; alkaline solution absorption; absorption or adsorption on activated carbon, ion-exchange resin or molecular sieve; modification of the solution pH and/or oxidation-reduction potential, evaporators, fractional crystallizers, solid/liquid separators, nanofiltration, and all combinations thereof.

Following the recovery of useful or valuable products from the process stream, according to certain embodiments, the removal of the waste products may be performed as indicated by the box 8. labeled "Waste removal" in FIG. 1. The remaining broth may be returned to the culture vessel along with replacement water and nutrients, if desired [see the process arrow labeled "Recycled H₂O + nutrients" in FIG. 1].

In certain embodiments of the present invention there is an acid co-product of chemosynthesis. Neutralization of acid in the broth can be accomplished in certain embodiments by the addition of bases including but not limited to: limestone, lime, sodium hydroxide, ammonia, caustic potash, magnesium oxide, iron oxide. In certain embodiments, the base may be produced from a carbon dioxide emission-free source such as naturally occurring basic minerals including but not limited to calcium oxide, magnesium oxide, iron oxide, iron ore, olivine containing a metal oxide, serpentine containing a metal oxide, ultramafic deposits containing metal oxides, and underground basic saline aquifers.

In addition to carbon dioxide captured through the chemosynthetic fixation of carbon, additional carbon dioxide can be captured and converted to carbonates or biominerals through the catalytic action of *Ralstonia sp.* microorganisms in certain embodiments of the present invention. For embodiments of the invention that augment the carbon captured through chemosynthesis with biocatalyzed mineral carbon sequestration, the use of *Ralstonia sp.* microorganisms capable of withstanding a high pH solution where carbon dioxide is thermodynamically favored to precipitate as carbonate may be advantageous in certain cases. Any carbonate or biomineral precipitate produced may be removed periodically or continuously from the system using, for example, solid/liquid separation techniques known in the art of process engineering.

An additional feature of certain embodiments of the present invention relates to the uses of chemical products generated through the chemosynthetic carbon capture and fixation process of certain embodiments of the invention. The chemical products of certain embodiments of the present invention can be applied to uses including but not limited to the following: in the production of fertilizers;.

An additional feature of certain embodiments of the present invention relates to the uses of biochemicals or biomass produced through the chemosynthetic process step or steps of certain embodiments of the present invention. Uses of the biomass product include: as a carbon source for large scale fermentations to produce produce various chemicals including but not limited to commercial enzymes, antibiotics, amino acids, vitamins, bioplastics, glycerol, or 1,3-propanediol; as a nutrient source for the growth of other microbes or organisms; as feed for animals including but not limited to cattle, sheep, chickens, pigs, or fish; as fertilizer; as sources of pharmaceutical, medicinal or nutritional substances; soil additives and soil stabilizers.

In order to give specific examples of the overall biological and chemical process for using chemoautotrophic microorganisms to capture CO₂ and produce biomass and other useful co-products, a number of process flow diagrams describing various embodiments of the present invention are now described. These specific examples should not be construed as limiting the present invention in any way and are provided for the sole purpose of illustration.

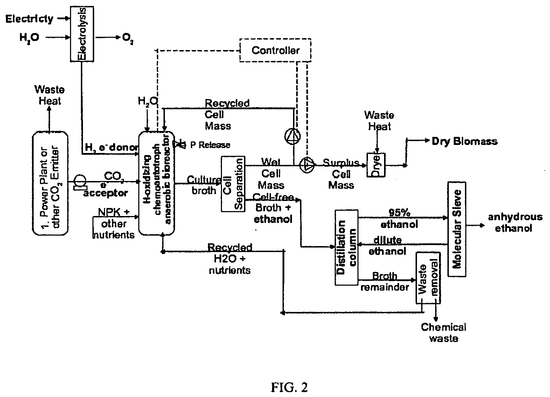

FIG. 2 is process flow diagram for an exemplary embodiment of the present invention for the capture of CO₂ by hydrogen oxidizing chemoautotrophs and production of ethanol. A carbon dioxide rich flue gas is captured from an emission source such as a power plant, refinery, or cement producer. The flue gas is then compressed and pumped into cylindrical anaerobic digesters containing one or more hydrogen oxidizing acetogenic chemoautotrophs such as but not limited to the following comparative chemoautotrophs: *Acetoanaerobium noterae, Acetobacterium woodii, Acetogenium kivui, Butyribacterium methylotrophicum, Butyribacterium rettgeri, Clostridium aceticum, Clostridium acetobutylicum, Clostridium acidi-urici, Clostridium autoethanogenum, Clostridium carboxidivorans, Clostridium formicoaceticum, Clostridium kluyveri, Clostridium ljungdahlii, Clostridium thermoaceticum, Clostridium thermoautotrophicum, Clostridium thermohydrosulfuricum, Clostridium thermosaccharolyticum, Clostridium thermocellum, Eubacterium limosum, Peptostreptococcus productus.* Hydrogen electron donor is added continuously to the growth broth along with other nutrients required for chemoautotrophic growth and maintenance that are pumped into the digester. In certain embodiments, the hydrogen source is a carbon dioxide emission-free process. This could be electrolytic or thermochemical processes powered by energy technologies including but not limited to photovoltaics, solar thermal, wind power, hydroelectric, nuclear, geothermal, enhanced geothermal, ocean thermal, ocean wave power, tidal power. Carbon dioxide serves as an electron acceptor in the chemosynthetic reaction. The culture broth is continuously removed from the digesters and flowed through membrane filters to separate the cell mass from the broth. The cell mass is then either recycled back into the digesters or pumped to driers depending upon the cell density in the digesters which is monitored by a controller. Cell mass directed to the dryers is then centrifuged and dried with evaporation. The dry biomass product is collected from the dryers. Cell-free broth which has passed through the cell mass removing filters is directed to vessels where the ethanol product is distilled and put through a molecular sieve to produce anhydrous ethanol using standard techniques known in the art of distillation. The broth left over after distillation is then subjected to any desired additional waste removal treatments which depends on the source of flue gas. The remaining water and nutrients are then pumped back into the digesters.

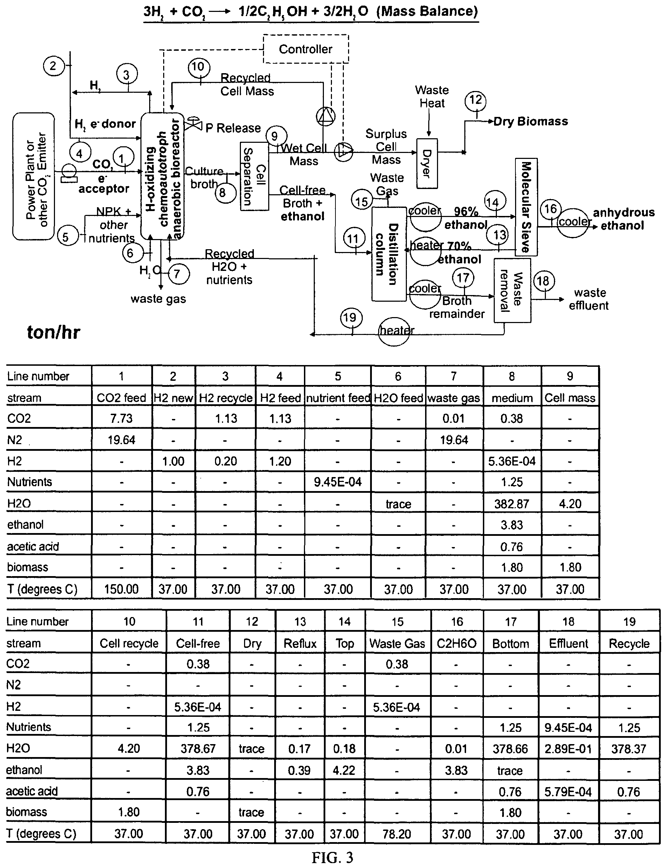

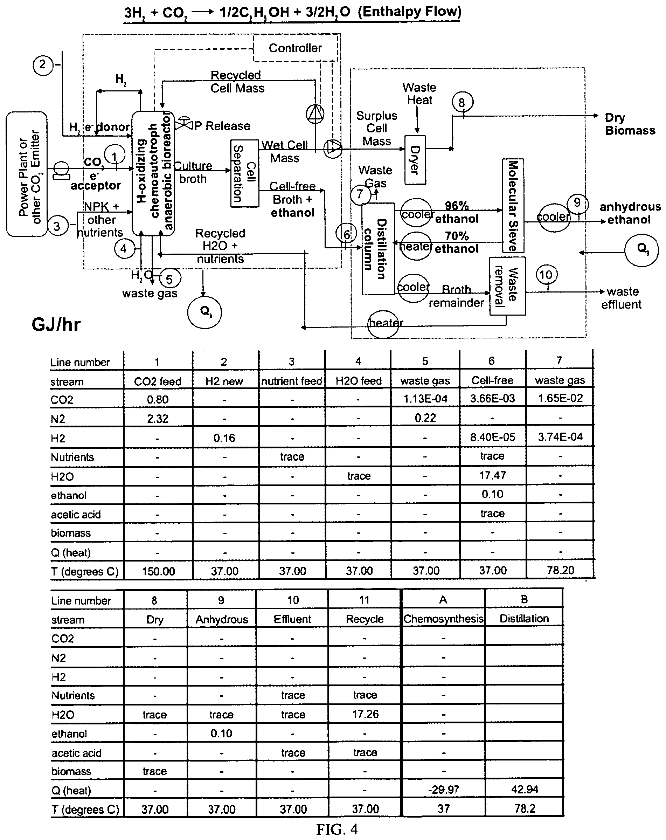

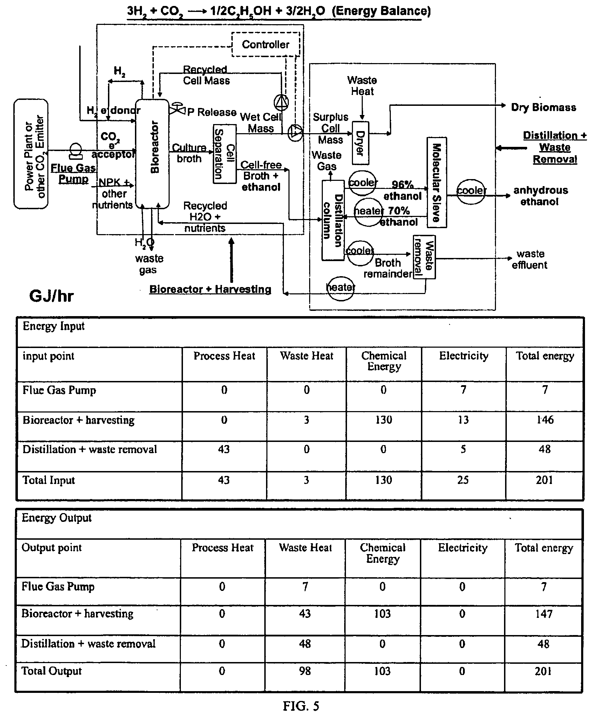

A process model is given in FIG. 3, 4 and 5 for the embodiment of FIG. 2. The mass balance, enthalpy flow, energy balance, and plant economics have been calculated for this [R.K. Sinnott, Chemical Engineering Design volume 6, 4th ed. (Elsevier Butterworth-Heinemann, Oxford, 2005)] preferred embodiment for the present invention. The model was developed using established results in the scientific literature for the H₂ oxidizing acetogens and for the process steps known from the art of chemical engineering. The inputs for the model regarding microorganism performance taken from the scientific literature [Gaddy, James L., et al. "Methods for increasing the production of ethanol from microbial fermentation". US Patent 7285402. Oct. 23 2007; Lewis, Randy S., et al. "Indirect or direct fermentation of biomass to fuel alcohol". US Patent Application 20070275447. Nov. 29 2007; Heiskanen, H., Virkajarvi, I., Viikari, L., 2007: The effect of syngas composition on the growth and product formation of Butyribacterium methylotrophicum. 41: 362-367] for acetogenic microorganisms were as follows: 1) stoichiometry of chemosynthetic reaction producing ethanol: 3H₂ + CO₂ -> 0.5C₂H₅OH + 1.5 H₂O; 2) conversion of H₂ each pass through bioreactor: 83%; 3) stoichiometry of acetic acid side reaction: 2H₂ + CO₂ -> 0.5C₂H₅OH + H₂O; 4) Cell growth rate in plateau phase steady state ~ 0; 5) percent of fixed carbon going to ethanol during steady state: 99.99%; 6) growth medium concentration of ethanol at steady state : 10 grams/liter; 7) ethanol productivity at steady state: 10 grams/liter/day; 8) concentration of acetic acid at steady state: 2 grams/liter; 9) cell mass concentration at steady state: 1.5 grams/liter. The mass balance indicates that 1 ton of ethanol will be produced for every 2 tons of CO₂ pumped into the system. This amounts to over 150 gallons of ethanol produced per ton of CO₂ intake. The energy balance indicates that for every 1 GJ of H₂ chemical energy input there is 0.8 GJ of ethanol chemical energy out, i.e. the chemical conversion is expected to be around 80% efficient. Overall efficiency of ethanol production from H₂ and CO₂ including electric power and process heat is predicted with the model to be about 50%.

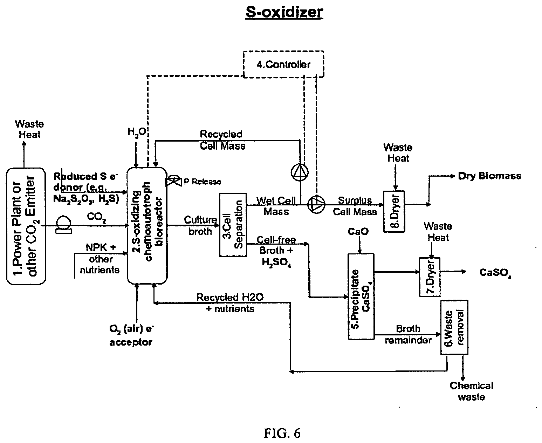

FIG. 6 is process flow diagram for a comparative process involving the capture of CO₂ by sulfur oxidizing chemoautotrophs and production of biomass and gypsum. A carbon dioxide rich flue gas is captured from an emission source such as a power plant, refinery, or cement producer. The flue gas is then compressed and pumped into cylindrical aerobic digesters containing one or more sulfur oxidizing chemoautotrophs such as but not limited to *Thiomicrospira crunogena, Thiomicrospira* strain MA-3, *Thiomicrospira thermophila, Thiobacillus hydrothermalis, Thiomicrospira sp.* strain CVO, *Thiobacillus neapolitanus, Arcobacter sp.* strain FWKO B. One or more electron donors such as but not limited to thiosulfate, hydrogen sulfide, or sulfur are added continuously to the growth broth along with other nutrients required for chemoautotrophic growth and air is pumped into the digester to provide oxygen as an electron acceptor. The culture broth is continuously removed from the digesters and flowed through membrane filters to separate the cell mass from the broth. The cell mass is then either recycled back into the digesters or pumped to driers depending upon the cell density in the digesters which is monitored by a controller. Cell mass directed to the dryers is then centrifuged and dried with evaporation. The dry biomass product is collected from the dryers. Cell-free broth which has passed through the cell mass removing filters is directed to vessels where the sulfuric acid produced by the chemosynthetic metabolism is neutralized with lime, precipitating out gypsum (CaSO₄). The lime may be produced in certain embodiments by a carbon dioxide emission-free process rather than through the heating of limestone. Such carbon dioxide emission-free processes include the recovery of natural sources of basic minerals including but not limited to minerals containing a metal oxide, serpentine containing a metal oxide, ultramafic deposits containing metal oxides, and underground basic saline aquifers. Alternative bases may be used for neutralization in this process including but not limited to magnesium oxide, iron oxide, or some other metal oxide. The gypsum is removed by solid-liquid separation techniques and pumped to dryers. The final product is dried gypsum. The broth left over after the sulfate is precipitated out is then subjected to any desired additional waste removal treatments which depends on the source of flue gas. The remaining water and nutrients are then pumped back into the digesters.

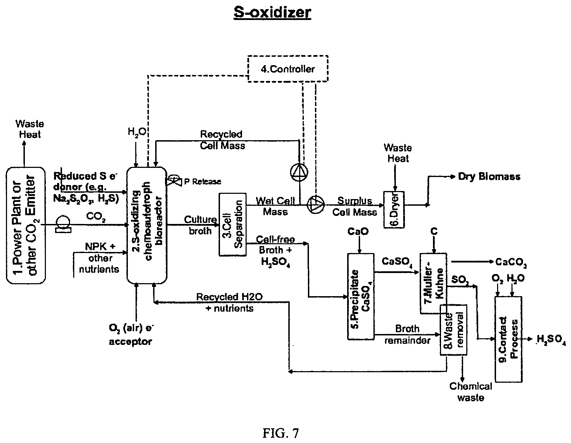

FIG. 7 is process flow diagram for a comparative process involving the capture of CO₂ by sulfur oxidizing chemoautotrophs and production of biomass and sulfuric acid and calcium carbonate via the Muller-Kuhne reaction. A carbon dioxide rich flue gas is captured from an emission source such as a power plant, refinery, or cement producer. The flue gas is then compressed and pumped into cylindrical aerobic digesters containing one or more sulfur oxidizing chemoautotrophs such as but not limited to *Thiomicrospira crunogena, Thiomicrospira* strain MA-3, *Thiomicrospira thermophila, Thiobacillus hydrothermalis, Thiomicrospira sp.* strain CVO, *Thiobacillus neapolitanus, Arcobacter sp.* strain FWKO B. One or more electron donors such as but not limited to thiosulfate, hydrogen sulfide, or sulfur are added continuously to the growth broth along with other nutrients required for chemoautotrophic growth and air is pumped into the digester to provide oxygen as an electron acceptor. The culture broth is continuously removed from the digesters and flowed through membrane filters to separate the cell mass from the broth. The cell mass is then either recycled back into the digesters or pumped to driers depending upon the cell density in the digesters which is monitored by a controller. Cell mass directed to the dryers is then centrifuged and dried with evaporation. The dry biomass product is collected from the dryers. Cell-free broth which has passed through the cell mass removing filters is directed to vessels where the sulfuric acid produced by the chemosynthetic metabolism is neutralized with lime (CaO), precipitating out gypsum (CaSO₄). The lime may be produced in certain embodiments by a carbon dioxide emission-free process rather than through the heating of limestone. Such carbon dioxide emission-free processes include the recovery of natural sources of basic minerals including but not limited to minerals containing a metal oxide, iron ore, serpentine containing a metal oxide, ultramafic deposits containing metal oxides, and underground basic saline aquifers. Alternative bases may be used for neutralization in this process including but not limited to magnesium oxide, iron oxide, or some other metal oxide. The gypsum is removed by solid-liquid separation techniques and pumped to kilns where the Muller-Kuhne process is carried out with the addition of coal. The net reaction for the Muller-Kuhne process is as follows 2C + 4CaSO₄ → 2CaO + 2CaCO₃ + 4SO₂. The produced CaCO₃ is collected and the CaO is recycled for further neutralization. The SO₂ gas produced is directed to a reactor for the contact process where sulfuric acid is produced. The broth left over after the sulfate is precipitated out is then subjected to any desired additional waste removal treatments which depends on the source of flue gas. The remaining water and nutrients are then pumped back into the digesters.

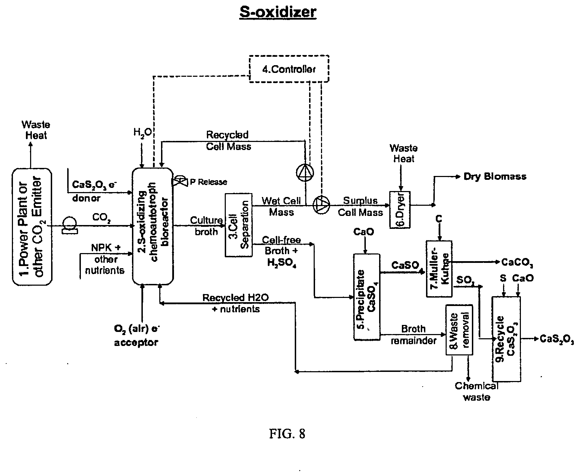

FIG. 8 is a process flow diagram for a comparative process involving the capture of CO₂ by sulfur oxidizing chemoautotrophs and production of biomass and calcium carbonate and recycling of thiosulfate electron donor via the Muller-Kuhne reaction. A carbon dioxide rich flue gas is captured from an emission source such as a power plant, refinery, or cement producer. The flue gas is then compressed and pumped into cylindrical aerobic digesters containing one or more sulfur oxidizing chemoautotrophs such as but not limited to *Thiomicrospira crunogena, Thiomicrospira* strain MA-3, *Thiomicrospira thermophila, Thiobacillus hydrothermalis, Thiomicrospira sp.* strain CVO, *Thiobacillus neapolitanus, Arcobacter sp.* strain FWKO B. Calcium thiosulfate is the electron donor added continuously to the growth broth along with other nutrients required for chemoautotrophic growth and air is pumped into the digester to provide oxygen as an electron acceptor. The culture broth is continuously removed from the digesters and flowed through membrane filters to separate the cell mass from the broth. The cell mass is then either recycled back into the digesters or pumped to driers depending upon the cell density in the digesters which is monitored by a controller. Cell mass directed to the dryers is then centrifuged and dried with evaporation. The dry biomass product is collected from the dryers. Cell-free broth which has passed through the cell mass removing filters is directed to vessels where the sulfuric acid produced by the chemosynthetic metabolism is neutralized with lime (CaO), precipitating out gypsum (CaSO₄). The lime may be produced in certain embodiments by a carbon dioxide emission-free process rather than through the heating of limestone. Such carbon dioxide emission-free processes include the recovery of natural sources of basic minerals including but not limited to minerals containing a metal oxide, serpentine containing a metal oxide, ultramafic deposits containing metal oxides, and underground basic saline aquifers. Alternative bases may be used for neutralization in this process including but not limited to magnesium oxide, iron oxide, or some other metal oxide. The gypsum is removed by solid-liquid separation techniques and pumped to kilns where the Muller-Kuhne process is carried out with the addition of coal. The net reaction for the Muller-Kuhne process is as follows 2C + 4CaSO₄ → 2CaO + 2CaCO₃ + 4SO₂. The produced CaCO₃ is collected and the CaO is recycled for further reaction. The SO₂ gas produced is directed to a reactor where it is reacted with CaO or some other metal oxide such as iron oxide, and sulfur to recycle the thiosulfate (calcium thiosulfate if CaO is used). The broth left over after the sulfate is precipitated out is then subjected to any desired additional waste removal treatments which depends on the source of flue gas. The remaining water and nutrients are then pumped back into the digesters.

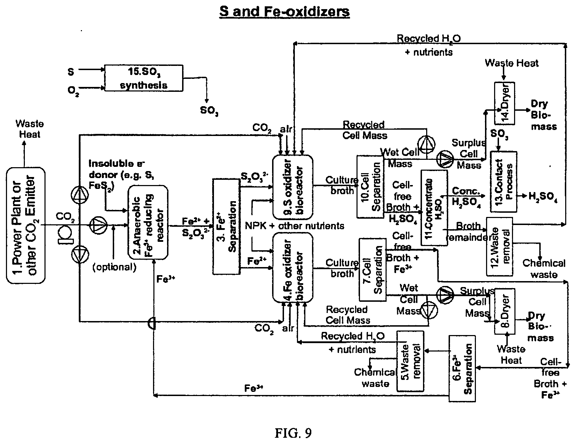

FIG. 9 is process flow diagram for a comparative process involving the capture of CO₂ by sulfur and iron oxidizing chemoautotrophs and production of biomass and sulfuric acid using an insoluble source of electron donors. A carbon dioxide rich flue gas is captured from an emission source such as a power plant, refinery, or cement producer. The flue gas is then compressed and pumped into one set of cylindrical aerobic digesters containing one or more sulfur oxidizing chemoautotrophs such as but not limited to *Thiomicrospira crunogena, Thiomicrospira* strain MA-3, *Thiomicrospira thermophila, Thiobacillus hydrothermalis, Thiomicrospira sp.* strain CVO, *Thiobacillus neapolitanus, Arcobacter sp.* strain FWKO B, and another set of cylindrical aerobic digesters containing one or more iron oxidizing chemoautotrophs such as but not limited to *Leptospirillum ferrooxidans* or *Thiobacillus ferrooxidans.* One or more insoluble sources of electron donors such as but not limited to elemental sulfur, pyrite, or other metal sulfides are sent to a anaerobic reactor for reaction with a ferric iron solution. Optionally chemoautotrophs such as but not limited to *Thiobacillus ferrooxidans* and *Sulfolobus sp.* can be present in this reactor to help biocatalyze the attack of the insoluble electron donor source with ferric iron. A leachate of ferrous iron and thiosulfate flow out of the reactor. The ferrous iron is separated out of the process stream by precipitation. The thiosulfate solution is then flowed into the S-oxidizer digesters and the ferrous iron is pumped into the Fe-oxidizer digesters as the electron donor for each type of chemoautotroph respectively. Air and other nutrients required for chemoautotrophic growth are also pumped into the digesters. The culture broth is continuously removed from the digesters and flowed through membrane filters to separate the cell mass from the broth. The cell mass is then either recycled back into the digesters or pumped to driers depending upon the cell density in the digesters which is monitored by a controller. Cell mass directed to the dryers is then centrifuged and dried with evaporation. The dry biomass product is collected from the dryers. In the S-oxidizer process stream the cell-free broth which has passed through the cell mass removing filters is directed to sulfuric acid recovery systems such employed in the refinery or distillery industries where the sulfuric acid product of chemosynthetic metabolism is concentrated. This sulfuric acid concentrate is then concentrated further using the contact process to give a concentrated sulfuric acid product. The broth left over after the sulfate and sulfuric acid have been removed is then subjected to any desired additional waste removal treatments which depends on the source of flue gas. In the Fe-oxidizer process stream the cell-free broth which has passed through the cell mass removing filters is then stripped of ferric iron by precipitation. This ferric iron is then sent back for further reaction with the insoluble source of electron donors (e.g. S, FeS₂). The remaining water and nutrients in both process streams are then pumped back into their respective digesters.

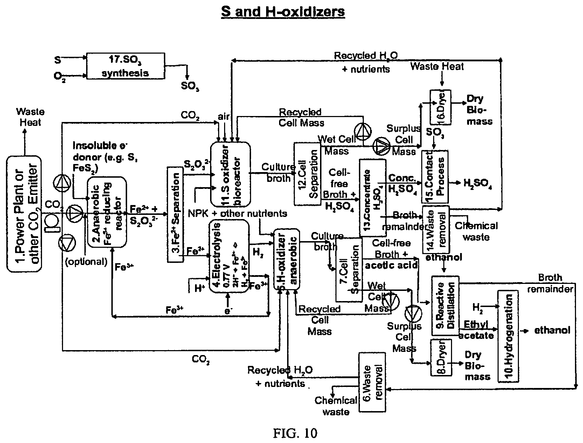

FIG. 10 is a process flow diagram for a comparative process involving the capture of CO₂ by sulfur and hydrogen oxidizing chemoautotrophs and production of biomass, sulfuric acid, and ethanol using an insoluble source of electron donors. A carbon dioxide rich flue gas is captured from an emission source such as a power plant, refinery, or cement producer. The flue gas is then compressed and pumped into one set of cylindrical aerobic digesters containing one or more sulfur oxidizing chemoautotrophs such as but not limited to *Thiomicrospira crunogena, Thiomicrospira* strain MA-3, *Thiomicrospira thermophila, Thiobacillus hydrothermalis, Thiomicrospira sp.* strain CVO, *Thiobacillus neapolitanus, Arcobacter sp.* strain FWKO B, and another set of cylindrical anaerobic digesters containing one or more hydrogen oxidizing acetogenic chemoautotrophs such as but not limited to *Acetoanaerobium noterae, Acetobacterium woodii, Acetogenium kivui, Butyribacterium methylotrophicum, Butyribacterium rettgeri, Clostridium aceticum, Clostridium acetobutylicum, Clostridium acidi-urici, Clostridium autoethanogenum, Clostridium carboxidivorans, Clostridium formicoaceticum, Clostridium kluyveri, Clostridium ljungdahlii, Clostridium thermoaceticum, Clostridium thermoautotrophicum, Clostridium thermohydrosulfuricum, Clostridium thermosaccharolyticum, Clostridium thermocellum, Eubacterium limosum, Peptostreptococcus productus.* One or more insoluble sources of electron donors such as but not limited to elemental sulfur, pyrite, or other metal sulfides are sent to an anaerobic reactor for reaction with a ferric iron solution. Optionally chemoautotrophs such as but not limited to *Thiobacillus ferrooxidans* and *Sulfolobus sp.* can be present in this reactor to help biocatalyze the attack of the insoluble electron donor source with ferric iron. A leachate of ferrous iron and thiosulfate flow out of the reactor. The ferrous iron is separated out of the process stream by precipitation. The thiosulfate solution is then flowed into the S-oxidizer digesters as an electron donor and the ferrous iron is pumped into an anaerobic electrolysis reactor. In the electrolysis reactor hydrogen gas is formed by the electrochemical reaction 2H⁺ + Fe²⁺ → H₂ + Fe³⁺. The open cell voltage for this reaction is 0.77 V which is substantially lower than the open cell voltage for the electrolysis of water (1.23 V). Furthermore the kinetics of the oxidation of ferrous iron to ferric iron is much simpler than that for the reduction of oxygen in water to oxygen gas, hence the overvoltage for the iron reaction is lower. These factors combined provides an energy savings for the production of hydrogen gas by using ferrous iron compared to electrolysis of water. The hydrogen produced is fed into the H-oxidizer digesters as the electron donor. The other nutrients required for chemoautotrophic growth are also pumped into the digesters. The culture broth is continuously removed from the digesters and flowed through membrane filters to separate the cell mass from the broth. The cell mass is then either recycled back into the digesters or pumped to driers depending upon the cell density in the digesters which is monitored by a controller. Cell mass directed to the dryers is then centrifuged and dried with evaporation. The dry biomass product is collected from the dryers. In the S-oxidizer process stream the cell-free broth which has passed through the cell mass removing filters is directed to sulfuric acid recovery systems such as employed in the refinery and distillation industries where the sulfuric acid product of chemosynthetic metabolism is concentrated. This sulfuric acid concentrate is then concentrated further using the contact process to give a concentrated sulfuric acid product. The broth left over after the sulfate and sulfuric acid have been removed is then subjected to any desired additional waste removal treatments which depends on the source of flue gas. In the H-oxidizer process stream the cell-free broth which has passed through the cell mass removing filters is directed to vessels where the acetic acid produced is reacted with ethanol to produce ethyl acetate which is removed from solution by reactive distillation. The ethyl acetate is converted to ethanol by hydrogenation. Part, e.g. half, of the ethanol is recycled for further reaction in the reactive distillation process. The other part is put through a molecular sieve which separates anhydrous ethanol by adsorbtion from dilute ethanol. The anhydrous ethanol is then collected and the dilute ethanol is returned for further reaction in the reactive distillation step. The broth left over after the acetic acid is reactively distilled out is then subjected to any desired additional waste removal treatments which depends on the source of flue gas. The remaining water and nutrients in both process streams are then pumped back into their respective digesters.

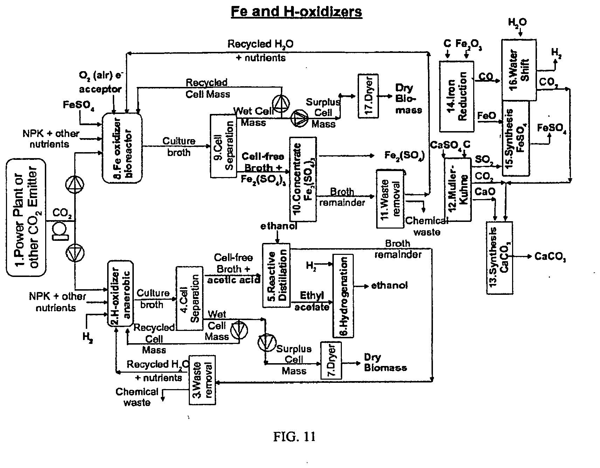

FIG. 11 is process flow diagram for a comparative process involving the capture of CO₂ by iron and hydrogen oxidizing chemoautotrophs and production of biomass, ferric sulfate, calcium carbonate and ethanol using coal or another hydrocarbon as the energy input for the production of electron donors without the release of gaseous CO₂. A carbon dioxide rich flue gas is captured from an emission source such as a power plant, refinery, or cement producer. The flue gas is then compressed and pumped into one set of cylindrical aerobic digesters containing one or more iron oxidizing chemoautotrophs such as but not limited to *Leptospirillum ferrooxidans* or *Thiobacillus ferrooxidans,* and another set of cylindrical anaerobic digesters containing one or more hydrogen oxidizing acetogenic chemoautotrophs such as but not limited to *Acetoanaerobium noterae, Acetobacterium woodii, Acetogenium kivui, Butyribacterium methylotrophicum, Butyribacterium rettgeri, Clostridium aceticum, Clostridium acetobutylicum, Clostridium acidi-urici, Clostridium autoethanogenum, Clostridium carboxidivorans, Clostridium formicoaceticum, Clostridium kluyveri, Clostridium ljungdahlii, Clostridium thermoaceticum, Clostridium thermoautotrophicum, Clostridium thermohydrosulfuricum, Clostridium thermosaccharolyticum, Clostridium thermocellum, Eubacterium limosum, Peptostreptococcus productus.* Hydrogen gas produced by the water shift reaction is fed into the H-oxidizer digesters as the electron donor. Ferrous sulfate synthesized through the reaction of ferrous oxide (FeO), sulfur dioxide and oxygen is pumped into the Fe-oxidizer digesters as the electron donor. The other nutrients required for chemoautotrophic growth are also pumped into the digesters for each respective type of chemoautotroph. The culture broth is continuously removed from the digesters and flowed through membrane filters to separate the cell mass from the broth. The cell mass is then either recycled back into the digesters or pumped to driers depending upon the cell density in the digesters which is monitored by a controller. Cell mass directed to the dryers is then centrifuged and dried with evaporation. The dry biomass product is collected from the dryers. In the Fe-oxidizer process stream the cell-free broth which has passed through the cell mass removing filters is directed to ferric sulfate recovery systems such as employed in the steel industry where the ferric sulfate product of chemosynthetic metabolism is concentrated into a salable product. The broth left over after the sulfate has been removed is then subjected to any desired additional waste removal treatments which depends on the source of flue gas. In the H-oxidizer process stream the cell-free broth which has passed through the cell mass removing filters is directed to vessels where the acetic acid produced is reacted with ethanol to produce ethyl acetate which is removed from solution by reactive distillation. The ethyl acetate is converted to ethanol by hydrogenation. Part, e.g. half, of the ethanol is recycled for further reaction in the reactive distillation process. The other part of the ethanol is put through a molecular sieve which separates anhydrous ethanol by adsorbtion from dilute ethanol. The anhydrous ethanol is then collected and the dilute ethanol is returned for further reaction in the reactive distillation step. The broth left over after the acetic acid is reactively distilled out is then subjected to any desired additional waste removal treatments which depends on the source of flue gas. The remaining water and nutrients in both process streams are then pumped back into their respective digesters. Both the hydrogen gas and ferrous sulfate electron donors are ultimately generated through the oxidation of coal or some other hydrocarbon. The oxidation drives two reactions that occur in parallel, one is the reduction of iron ore (Fe₂O3) to ferrous oxide (FeO) accompanied by the release of carbon monoxide which is water shifted to produce hydrogen gas and carbon dioxide, the other is the reduction of gypsum (CaSO₄) to sulfur dioxide and quicklime accompanied by the release of carbon dioxide. The carbon dioxide from both process streams is reacted with the quicklime to produce calcium carbonate. In parallel with the production of calcium carbonate is the production of ferrous sulfate through the reaction of ferrous oxide with sulfur dioxide and oxygen.

It should be noted that in all of the previously described comparative processes with a sulfuric acid product the sulfuric acid may alternatively be neutralized, in certain embodiments with a base that is not a carbonate (so as to not release carbon dioxide in the acid base reaction) and this carbonate may be produced by a carbon dioxide emission-free process. Such bases include but are not limited to natural basic minerals containing a metal oxide, serpentine containing a metal oxide, ultramafic deposits containing metal oxides, underground basic saline aquifers, and naturally occurring calcium oxide, magnesium oxide, iron oxide, or some other metal oxide. The metal sulfate which results from the acid-base reaction may be recovered from the process stream and preferably refined into a salable product, while the water produced by the acid-base reaction may be recycled back into the chemosynthesis reactors.

The following comparative example is intended to illustrate certain features or advantages of the present invention.

### Comparative Example

A specific comparative example is provided to demonstrate the carbon capture and fixation capabilities of chemoautotrophic microorganisms that play a central part in the overall carbon capture and fixation process of the present invention.

Tests were performed on the sulfur-oxidizing chemoautotroph Thiomicrospira crunogena ATCC #35932 acquired as a freeze dried culture from American Type Culture Collection (ATCC). The organisms were grown on the recommended ATCC medium - the #1422 broth. This broth consisted of the following chemicals dissolved in 1 Liter of distilled water:
NaCl, 25.1g; (NH₄)₂SO₄, 1.0g; MgSO₄.7 H₂O, 1.5g; KH₂PO₄, 0.42g; NaHCO₃, 0.20g; CaCl₂.2 H₂O, 0.29g; Tris-hydrochloride buffer, 3.07g; Na₂S₂O₃.5H₂O, 2.48g; Visniac and Santer Trace Element Solution, 0.2 ml; 0.5% Phenol Red, 1.0 ml;

The #1422 broth was adjusted to pH 7.5 and filter-sterilized prior to innoculation.

The freeze dried culture of Thiomicrospira crunogena was rehydrated according to the procedure recommended by ATCC and transferred first to a test tube with 5 ml broth #1422 and placed on a shaker. This culture was used to innoculate additional test tubes. NaOH was added as needed to maintain the pH near 7.5. Eventually the cultures were transferred from the test tube to 1 liter flasks filled with 250 ml of #1422 broth and placed in a New Brunswick Scientific Co. shake flask incubator set to 25 Celsius.

The determination of growth rate for Thiomicrospira crunogena was performed using the following procedure: 1) Three (1 litre) flasks containing 95 ml ATCC 1422 medium were innoculated with 5 ml of the above cultures diluted to an optical density ~0.025. Optical densities were determined using a Milton Roy Spectronic 1001 Spectrophotometer; 2) Two ml samples of cultures were withdrawn from each flask from t=0 to t=48 hours at every 2 hour intervals and optical density measured. Optical density was correlated with dry weight weighing twice centrifuged and washed, 1 mL liquid broth oven dried samples in pre-weighed aluminum dishes.

From the growth curve is was found that in the exponential phase the doubling time for *Thiomicrospira crunogena* was one hour. This is about 4 to 6 times shorter doubling time than the fastest growth rates reported for algae in the exponential phase [Sheehan et al, 1998, "A Look Back at the U.S. Department of Energy's Aquatic Species Program―Biodiesel from Algae"]. The cell mass density present in the flask experiments when the microorganisms were in the exponential growth phase reached 0.5 g dry weight /liter, and in the plateau phase the cell mass density reached 1 g dry weight/liter. This indicates that in a continuous system that maintains the culture in the exponential growth state with continuous cell removal, these microorganisms have the potential to produce 12 g dry weight / liter/ day of biomass. This is about 4-12 times faster than the highest daily rates of biomass production reported for algae [Valcent, 2007; CNN, 2008]. Furthermore, in a continuous bioreactor substantially higher cell densities should be able to be sustained in the exponential phase than what can be achieved at the flask level with *T. crunogena.* This experiment supports the far higher rates of carbon fixation that are attainable with chemoautotrophic than photosynthetic microbes.

The indefinite articles "a" and "an," as used herein in the specification and in the claims, unless clearly indicated to the contrary, should be understood to mean "at least one."
The phrase "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, in one embodiment, to A without B (optionally including elements other than B); in another embodiment, to B without A (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

In the claims, as well as in the specification above, all transitional phrases such as "comprising," "including," "carrying," "having," "containing," "involving," "holding," and the like are to be understood to be open-ended, i.e., to mean including but not limited to. Only the transitional phrases "consisting of' and "consisting essentially of' shall be closed or semi-closed transitional phrases, respectively.

## Claims

1. A biological and chemical method for the capture and conversion of carbon dioxide into organic compounds, comprising:
introducing carbon dioxide gas, either alone and/or dissolved in a mixture or solution further comprising carbonate ion and/or bicarbonate ion into an environment suitable for maintaining chemoautotrophic organisms and/or chemoautotroph cell extracts; and
fixing the carbon dioxide and/or inorganic carbon into organic compounds within the environment via at least one chemosynthetic carbon fixing reaction utilizing chemoautotrophic microorganisms comprising a *Ralstonia sp.;*
wherein where the chemosynthetic carbon fixing reaction is driven by chemical and/or electrochemical energy provided by electron donors and electron acceptors that have been generated chemically and/or electrochemically and/or are introduced into the environment from at least one source external to the environment, and wherein the electron donor comprises hydrogen generated through an electrolysis method selected from the group comprising proton exchange membrane electrolysis, liquid electrolyte electrolysis, high pressure electrolysis, and high temperature electrolysis of steam and said electron acceptor comprises carbon dioxide and/or oxygen;
wherein the environment suitable for maintaining chemoautotrophic organisms and/or chemoautotroph cell extracts is maintained using continuous influx and removal of nutrient medium and/or biomass, in substantially steady state where the cell population and environmental parameters are targeted at a substantially constant suitable or optimal level over time; and
wherein biomass is produced by the at least one chemosynthetic reaction, and wherein the biomass is separated from the environment and is processed into a product comprising an animal feed, a fertilizer, a soil additive, a soil stabilizer, a carbon source for large scale fermentations, a nutrient source for the growth of other microbes or organisms, and/or as a source of pharmaceutical, medicinal or nutritional substances.

2. A method according to Claim 1, wherein fixing step is followed by one or more process steps in which organic and/or inorganic chemical products of chemosynthesis are separated from a process stream produced during the fixing step and processed to form products in a form suitable for storage, shipping, and sale.

3. A method according to any preceding claim, wherein said electron donors and/or said electron acceptors are generated or recycled using renewable, alternative, or conventional sources of power that are low in greenhouse gas emissions, and wherein said sources of power are selected from at least one of photovoltaics, solar thermal, wind power, hydroelectric, nuclear, geothermal, enhanced geothermal, ocean thermal, ocean wave power, tidal power, and carbon capture and sequestration enabled methane reforming, or carbon capture and sequestration enabled gasification or pyrolysis of coal or biomass.

4. A method according to any preceding claim, further comprising reacting carbon dioxide with minerals to form a carbonate or bicarbonate product.

5. A method according to any preceding claim, wherein carbon dioxide is introduced in the introducing step, and wherein the carbon dioxide is dissolved in aqueous solution.

6. A method according to any preceding claim, wherein molecular hydrogen acts as an electron donor and wherein hydrogen concentrations in air between 4 to 74.5% are avoided.

7. A method according to any preceding claim, whereby the culture broth is continuously removed from the environment suitable for maintaining chemoautotrophic organisms and flowed through membrane filters to separate the cell mass from the broth.

8. A method according to any preceding claim, wherein the biomass produced by the at least one chemosynthetic reaction is centrifuged and then dried with evaporation, and where the biomass product is collected from the dryers.

9. The method according to claim 7 or 8 where remaining water and nutrients separated from the biomass are pumped back into the environment suitable for maintaining chemoautotrophic organisms.

## Patentansprüche

1. Biologisches und chemisches Verfahren zum Abscheiden und Umwandeln von Kohlendioxid in organische Verbindungen, das Folgendes beinhaltet:
Einleiten von Kohlendioxidgas, entweder allein und/oder in einem Gemisch oder einer Lösung gelöst, das ferner Carbonationen und/oder Bicarbonationen enthält, in eine Umgebung, die zum Halten von chemoautotrophen Organismen und/oder chemoautotrophen Zellextrakten geeignet ist; und
Fixieren des Kohlendioxids und/oder anorganischen Kohlenstoffs in organische Verbindungen innerhalb der Umgebung über mindestens eine chemosynthetische Kohlenstofffixierungsreaktion unter Verwendung von chemoautotrophen Mikroorganismen, die eine *Ralstonia sp.* umfassen;
wobei die chemosynthetische Kohlenstofffixierungsreaktion durch chemische und/oder elektrochemische Energie angetrieben wird, die durch Elektronendonatoren und Elektronenakzeptoren bereitgestellt wird, die chemisch und/oder elektrochemisch erzeugt wurden und/oder in die Umgebung aus mindestens einer Quelle außerhalb der Umgebung eingeleitet werden, und wobei der Elektronendonator Wasserstoff umfasst, der durch ein Elektrolyseverfahren erzeugt wird, ausgewählt aus der Gruppe, die Protonenaustauschmembranelektrolyse, Flüssigelektrolytelektrolyse, Hochdruckelektrolyse und Hochtemperaturelektrolyse von Dampf umfasst, und der Elektronenakzeptor Kohlendioxid und/oder Sauerstoff umfasst;
wobei die zum Halten von chemoautotrophen Organismen und/oder chemoautotrophen Zellextrakten geeignete Umgebung mittels eines kontinuierlichen Zuflusses und Abflusses von Nährmedium und/oder Biomasse in einem im Wesentlichen stabilen Zustand gehalten wird, in dem die Zellpopulation und die Umgebungsparameter auf ein im Wesentlichen konstantes geeignetes oder optimales Niveau über die Zeit hinweg ausgerichtet sind; und
wobei Biomasse durch die mindestens eine chemosynthetische Reaktion erzeugt wird und wobei die Biomasse von der Umwelt abgetrennt und zu einem Produkt verarbeitet wird, das ein Tierfutter, ein Düngemittel, einen Bodenzusatz, einen Bodenstabilisator, eine Kohlenstoffquelle für Großfermentationen, eine Nährstoffquelle für das Wachstum anderer Mikroben oder Organismen und/oder als Quelle für pharmazeutische, medizinische oder ernährungsphysiologische Substanzen umfasst.

2. Verfahren nach Anspruch 1, wobei auf den Fixierungsschritt ein oder mehrere Verfahrensschritte folgen, in denen organische und/oder anorganische chemische Chemosyntheseprodukte von einem während des Fixierungsschritts erzeugten Prozessstrom abgetrennt und zu Produkten in einer für Lagerung, Versand und Verkauf geeigneten Form verarbeitet werden.

3. Verfahren nach einem vorherigen Anspruch, wobei die genannten Elektronendonatoren und/oder die genannten Elektronenakzeptoren mittels erneuerbarer, alternativer oder konventioneller Energiequellen, die geringe Treibhausgasemissionen aufweisen, erzeugt oder recycelt werden, und wobei die genannten Energiequellen aus mindestens einem von Photovoltaik, Solarthermie, Windenergie, Wasserkraft, Kernenergie, Geothermie, verstärkter Geothermie, thermischer Meeresenergie, Meereswellenenergie, Gezeitenkraft und durch Kohlenstoffabscheidung und -sequestrierung ermöglichte Methanreformierung oder durch Kohlenstoffabscheidung und -sequestrierung ermöglichte Vergasung oder Pyrolyse von Kohle oder Biomasse ausgewählt werden.

4. Verfahren nach einem vorherigen Anspruch, das ferner das Umsetzen von Kohlendioxid mit Mineralien zur Bildung eines Carbonat- oder Bicarbonatprodukts beinhaltet.

5. Verfahren nach einem vorherigen Anspruch, wobei Kohlendioxid im Einleitungsschritt eingeleitet wird und wobei das Kohlendioxid in einer wässrigen Lösung gelöst ist.

6. Verfahren nach einem vorherigen Anspruch, wobei molekularer Wasserstoff als Elektronendonator wirkt und Wasserstoffkonzentrationen in Luft zwischen 4 und 74,5 % vermieden werden.

7. Verfahren nach einem vorherigen Anspruch, wobei die Kulturbrühe kontinuierlich aus der zum Halten chemoautotropher Organismen geeigneten Umgebung entfernt und durch Membranfilter geleitet wird, um die Zellmasse von der Brühe zu trennen.

8. Verfahren nach einem vorherigen Anspruch, wobei die durch die mindestens eine chemosynthetische Reaktion erzeugte Biomasse zentrifugiert und dann durch Verdampfen getrocknet wird, und wobei das Biomasseprodukt aus den Trocknern gesammelt wird.

9. Verfahren nach Anspruch 7 oder 8, wobei das/die von der Biomasse abgetrennte(n) restliche(n) Wasser und Nährstoffe zurück in die Umgebung gepumpt werden, die zum Halten chemoautotropher Organismen geeignet ist.

## Revendications

1. Procédé biologique et chimique pour le piégeage de dioxyde de carbone et sa conversion en composés organiques, le procédé consistant à :
introduire du dioxyde de carbone gazeux, seul et/ou dissous dans un mélange ou une solution comprenant en outre un ion carbonate et/ou un ion bicarbonate dans un environnement approprié pour conserver des organismes chimioautotrophes et/ou des extraits de cellules chimioautotrophes ; et
fixer le dioxyde de carbone et/ou le carbone inorganique dans des composés organiques dans l'environnement par l'intermédiaire d'au moins une réaction de fixation de carbone chimiosynthétique au moyen de micro-organismes chimioautotrophes comprenant une espèce de Ralstonia ;
la réaction de fixation de carbone chimiosynthétique étant entraînée par une énergie chimique et/ou électrochimique fournie par des donneurs d'électrons et des accepteurs d'électrons qui ont été générés chimiquement et/ou électrochimiquement et/ou qui sont introduits dans l'environnement à partir d'au moins une source externe à l'environnement, et le donneur d'électrons comprenant de l'hydrogène généré par un procédé d'électrolyse choisi dans le groupe constitué d'une électrolyse à membrane échangeuse de protons, d'une électrolyse à électrolyte liquide, d'une électrolyse à haute pression et d'une électrolyse à haute température de vapeur, et ledit accepteur d'électrons comprenant du dioxyde de carbone et/ou de l'oxygène ;
l'environnement approprié pour conserver des organismes chimioautotrophes et/ou des extraits de cellules chimioautotrophes étant maintenu, au moyen d'un apport et d'un retrait continus de milieu nutritif et/ou de biomasse, dans un état sensiblement stable dans lequel la population cellulaire et les paramètres environnementaux sont ciblés à un niveau approprié ou optimum sensiblement constant dans le temps ; et
la biomasse étant produite par l'au moins une réaction chimiosynthétique et la biomasse étant séparée de l'environnement et étant traitée dans un produit comprenant des aliments pour animaux, un engrais, un additif de sol, un stabilisateur de sol, une source de carbone pour des fermentations à grande échelle, une source nutritive pour la croissance d'autres microbes ou organismes et/ou une source de substances pharmaceutiques, médicales ou nutritionnelles.

2. Procédé selon la revendication 1, dans lequel l'étape de fixation est suivie d'une ou plusieurs étapes de traitement dans lesquelles des produits chimiques organiques et/ou inorganiques de chimiosynthèse sont séparés d'un flux de traitement produit pendant l'étape de fixation et traités pour former des produits sous une forme adaptée au stockage, à l'expédition et à la vente.

3. Procédé selon l'une quelconque des revendications précédentes, dans lequel lesdits donneurs d'électrons et/ou lesdits accepteurs d'électrons sont générés ou recyclés au moyen de sources d'énergie renouvelables, alternatives ou conventionnelles qui émettent peu de gaz à effet de serre, et dans lequel lesdites sources d'énergie sont choisies parmi au moins une des sources suivantes : photovoltaïque, thermosolaire, éolienne, hydroélectrique, nucléaire, géothermique, géothermique améliorée, thermique des mers, houlomotrice des mers, marémotrice et réformage du méthane compatible avec le piégeage et la séquestration du carbone ou bien gazéification ou pyrolyse du charbon et de la biomasse compatible avec le piégeage et la séquestration du carbone.

4. Procédé selon l'une quelconque des revendications précédentes, consistant en outre à faire réagir du dioxyde de carbone avec des minéraux pour former un produit de carbonate ou de bicarbonate.

5. Procédé selon l'une quelconque des revendications précédentes, dans lequel le dioxyde de carbone est introduit à l'étape d'introduction et dans lequel le dioxyde de carbone est dissous dans une solution aqueuse.

6. Procédé selon l'une quelconque des revendications précédentes, dans lequel l'hydrogène moléculaire agit comme un donneur d'électrons et dans lequel des concentrations d'hydrogène dans l'air comprises entre 4 et 74,5 % sont évitées.

7. Procédé selon l'une quelconque des revendications précédentes, dans lequel le bouillon de culture est retiré en continu de l'environnement approprié pour conserver des organismes chimioautotrophes et/ou mis en circulation à travers des filtres à membrane pour séparer la masse cellulaire du bouillon.

8. Procédé selon l'une quelconque des revendications précédentes, dans lequel la biomasse produite par l'au moins une réaction chimiosynthétique est centrifugée puis séchée par évaporation, et dans lequel le produit de biomasse est collecté à partir des sécheuses.

9. Procédé selon la revendication 7 ou 8, dans lequel l'eau et les nutriments restants séparés de la biomasse sont renvoyés vers l'environnement approprié pour conserver des organismes chimioautotrophes.
